# EUROPEAN PATENT APPLICATION

(11) **EP 4 585 156 A1**
(43) Date of publication of application: **16.07.2025**
(21) Application number: 24151689.7
(22) Date of filing: 12.01.2024
(51) Int. Cl.: A61B 6/03, A61B 6/04, A61B 6/00

(54) **SYSTEM AND METHOD FOR MONITORING A PATIENT**

(71) Applicant: Koninklijke Philips N.V., 5656 AG Eindhoven (NL)
(72) Inventor: FRERKING, Lena Christina, Eindhoven (NL); BYSTROV, Daniel, Eindhoven (NL); SAALBACH, Axel, 5656AG Eindhoven (NL); SENEGAS, Julien Thomas, Eindhoven (NL)
(74) Representative: Philips Intellectual Property & Standards

(57) **Abstract**

A system is provided for monitoring a patient on a patient support during a medical scan. A camera generates camera images of the patient as they move on the patient support. A first camera image is selected before a first scan and a second camera image is selected after the first scan and before a second scan. The second scan is planned based on the position of the patient during the first scan. Patient movement is detected between the first and second camera images and an indication of the patient movement is output, which indicates that the patient may not be correctly positioned for the second scan.

## Description

### FIELD OF THE INVENTION

This invention relates to the monitoring of a patient, during a medical scan, such as a CT scan.

### BACKGROUND OF THE INVENTION

For the acquisition of diagnostic CT images, surview images are acquired in advance. For this, the patient table is moved inside the CT scanner and then either a 3D scout image or one or two X-ray projections are acquired. These scout images or X-ray projections are then used to exactly define the 3D location of the subsequent diagnostic scan requiring that the patient does not move between the scans.

Even though patients are instructed not to move during the entire imaging procedure, motion is a common problem, which can have substantial impact on the quality of the final scan. Specifically, motion between the surview and the diagnostic scan could result in a misplaced field of view, which would limit the diagnostic value of the data (potentially requiring a re-scan). More generally, movement between multiple scans which are intended for the same patient position is problematic.

In recent years, more and more CT systems are equipped with camera-based solutions to automate various aspects of the image acquisition workflow. Next to conventional RGB cameras, often RGB-D cameras are employed, which additionally allow for the acquisition of depth information.

Most of these cameras are installed for the monitoring of the patient on the patient support (couch). For example, during a CT examination, the patient is typically left alone in the CT room until the completion of the exam. Even though CT scans are rather short in time, multiple scans can be done in sequence, with or without contrast agent administration, so that a typical examination can take up to 15 minutes. It is known to monitor the patient during this time to check that she or he feels safe and comfortable and can follow the instructions.

However, this monitoring is not able to detect patient movements between a multiple scans, such as a surview scan and a diagnostic scan.

### SUMMARY OF THE INVENTION

The invention is defined by the claims.

According to examples in accordance with an aspect of the invention, there is provided a system for monitoring a patient during a medical scan, wherein the medical scan involves moving the patient on a patient support relative to a scanning gantry, the system comprising:
a camera for generating camera images of the patient on the patient support; and
a processor for processing the camera images, wherein the processor is configured to:
   select a first camera image before a first scan;
   select a second camera image after the first scan and before a second scan;
   process the first and second camera images to detect patient movement between the first and second camera images; and
   output an indication of the patient movement.

This monitoring system compares images taken of the patient on the patient support just before a first scan and a just before a second scan, so that it can be determined if the patient has moved between the two images, i.e., between the two scans. In this way, it can be ensured that there is correct patient alignment between the scans. By "patient movement between the first and second camera images" is meant movement during the time between the time at which the first camera image was taken and the time at which the second camera image was taken.

In one example the first and second camera images are taken with the patient support at a same patient support position. Thus, images are chosen which relate to the same patient support position. This simplifies the image processing to detect patient movement.

In another example, the first and second camera images are taken with the patient support at different patient support positions. The absolute table position (its longitudinal position and its height) can be tracked by the internal table sensors, or derived from image processing. Hence, the positions of the subject corresponding to two images taken at different time points can be registered by correcting for the different patient support positions. Based on this registration, it can be assessed whether the patient moved relative to the patient support positions, i.e., "locally".

The first scan for example comprises a surview scan and the second scan comprises a diagnostic scan. Thus, the system is able to determine if the patient is suitably positioned for a diagnostic scan which has been planned based on the results of the surview scan. The camera images which are used are thus images of the patient shortly before the surview acquisition and shortly before the diagnostic scan. General movement, or even movements of the anatomical region of interest, may then be assessed. If any movements are detected, the system can for example warn the operator with an alarm or stop the acquisition, or estimate the motion and adapt the final position of the diagnostic scan.

In one example, the processor is configured to receive an input indicating the patient support position. Thus, the movement of the patient support may be monitored by patient support sensors, so that no additional processing is needed to track the patient support position.

In another example, the processor is configured to analyze the camera images to track patient support movement and thereby determine the patient support position. Thus, image analysis may be used to track patient support movement without needing input from the scanner itself.

In one example, the processor is configured to process the first and second camera images by registering the images to detect differences. These differences are then indicative of movement between the images.

In another example, the processor is configured to process the first and second camera images by finding a spatial transformation that maximizes an image similarity measure. This spatial transformation is then indicative of the movement.

In another example, the processor is configured to process the first and second camera images by performing a flow analysis between the images of parts thereof.

In another example, the processor is configured to process the first and second camera images by detecting landmark positions in the images.

Thus, there are various known ways to analyze images to detect overall movement, or to detect specific regions where there is movement (or just differences). One or more different analysis approaches may be used.

The indication of the patient movement for example comprises an alarm. This then allows a system operator to decide the action to be taken. As discussed below, automated actions may also be taken, to adapt the second scan to compensate for patient movement.

The invention also provides a medical scanner comprising;
a patient support;
a scanning gantry;
a drive system for moving the patient support relative to a scanning gantry in a patient movement direction;
   a scan controller for controlling the medical scanner to implement a scanning plan; and the monitoring system as define above.

The scan controller is for example configured to modify the scanning plan based on the indication of patient movement. The scanning plan includes the position of the region to be scanned relative to the scanning gantry. This provides automatic plan adjustment so that the second scan (e.g., diagnostic scan) correctly implements the plan based on the position of the patient during the first scan (e.g., surview scan).

The invention also provides a method for monitoring a patient during a medical scan, wherein the medical scan involves moving the patient on a patient support relative to a scanning gantry, the method comprising:
selecting a first camera image before a first scan;
selecting a second camera image after the first scan and before a second scan;
processing the first and second camera images to detect patient movement between the first and second camera images; and
outputting an indication of the patient movement.

The method may comprise taking the first and second camera images with the patient support at a same first patient support position. Alternatively, the method comprises taking the first and second camera images with the patient support at different patient support positions and processing the first and second camera images takes into account the patient support position. The mage processing may then take account of the different patient support positions.

The method may comprise receiving an input indicating the patient support position or analyzing the camera images to track patient support movement and thereby determine the patient support position.

Processing the first and second camera images may be by:
registering the images to detect differences; or
finding a spatial transformation that maximizes an image similarity measure; or
performing a flow analysis between the images or parts thereof; or
by detecting landmark positions in the images.

The invention also provides a computer program comprising computer program code which is adapted, when said program is run on a computer, to implement the method defined above.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 shows schematically an example of a medical scanner with an imaging system for capturing optical images of the patient during the medical scan;
Fig. 2 shows images taken at different times during a scan procedure involving two scans;
Fig. 3 shows how a scan plan is modified based on detected movement; and
Fig. 4 shows a method for monitoring a patient during a medical scan.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a system for monitoring a patient on a patient support during a medical scan. A camera generates camera images of the patient on the patient support. A first camera image is selected before a first scan and a second camera image is selected after the first scan and before a second scan. The second scan is planned based on the position of the patient during the first scan. Patient movement is detected between the first and second camera images and an indication of the patient movement is output, which indicates that the patient may not be correctly positioned for the second scan.

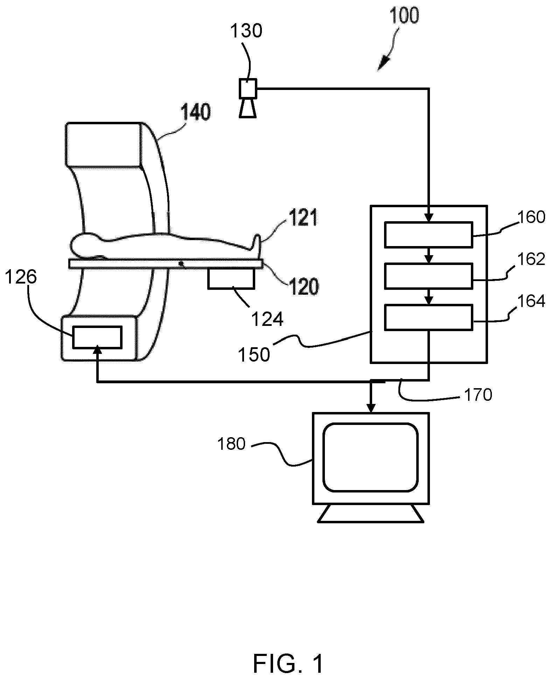

Fig. 1 shows schematically an example of a medical scanner 100 for acquiring a medical image of a subject and which additionally includes an imaging system for capturing optical images of the patient during the medical scan. The images are generated for the purposes of verifying a correct and static patient position between at least two sequential scans.

The medical scanner 100 comprises a scanning system such as a CT imaging system 140 that is adapted to acquire a medical image, i.e., a CT image in this example, of a patient 121 positioned on a patient support 120. The patient support 120 is adapted to move the patient 121 through the CT imaging system 140 during the CT imaging procedure. For this purpose, the medical scanner has a drive system 124 for driving the patient support through the scanning system.

A scan controller 126 controls the scanner to implement a desired workflow. The control involves at least defining the region to be scanned, as a volume in space relative to the coordinate system of the scanner.

The imaging system comprises an optical camera 130 that is adapted to acquire monitoring images of the patient 121 during the CT imaging procedure and a processor 150. The operation of the system with a single camera will be explained, but there may be multiple cameras. The camera can be a color or monochrome camera. It may be a 2D camera or a depth camera. It can use visible light or infrared light. The camera is calibrated with respect to the scanner, i.e., the scanner gantry.

The camera is mounted in a fixed position relative to the scanner. It is for example mounted on a ceiling of the scanning room.

The camera 130 has a wide field of view so that it captures a full view of the patient support 120, or at least the part of the patient support 120 over which regions of interest of the patient will be positioned.

The camera for example comprises a fisheye lens with a wide field of view. The patient support (and the patient on the patient support) can be imaged either by a single camera as shown or by a small set of cameras.

In accordance with the invention, the processor 150 selects a first camera image before a first scan. This function is represented by step 160. A second camera image is selected after the first scan and before a second scan. This function is represented by step 162.

The first and second camera images are processed to detect patient movement between the first and second camera images, as represented by step 164.

An indication of the patient movement 170 is then output. In the example of Fig. 1, the indication of patient movement is shown as being provided to a display terminal 180, for example as an alarm, and also to the scan controller 126. Either one, or both, of these options may be implemented.

In one example, the first and second camera images are taken at the same patient support position. In this way, all static parts of the system, as well as the patient support itself, will show no movement between the two images, hence simplifying the image analysis. However, the first and second images may be taken at different patient support positions. Because the patient support position (relative to the static parts of the imaging system) is known, the image processing can take into account this patient support position and thus perform image analysis accordingly. The patient support position may be known from sensor signals, or patient support drive signals, or from image analysis,

The patient support motion may correspond to a known vector direction in the captured images, so that a difference in patient support position will correspond to a shift in the image registration in that vector direction. Thus, the image processing may involve image transformation to realign the patient support between different images.

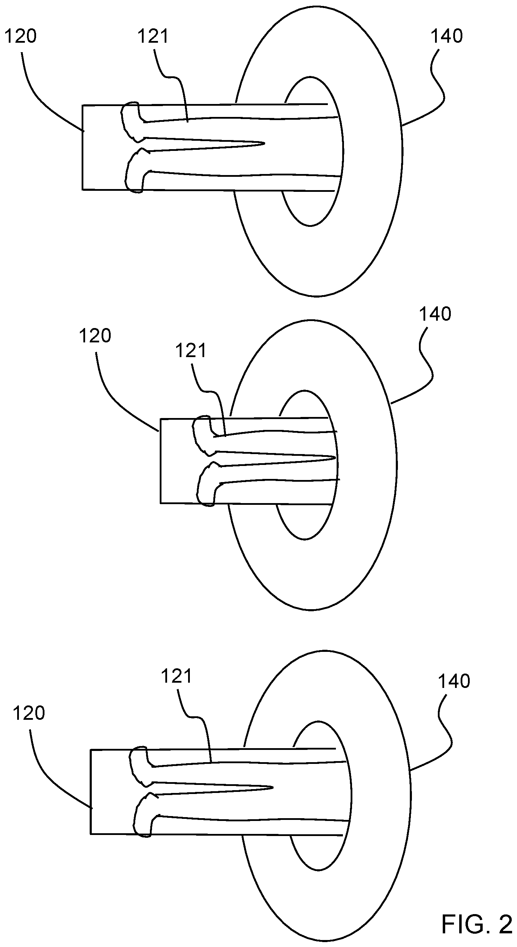

Fig. 2 depicts schematically three images from different steps in the imaging workflow using a ceiling mounted camera 130.

The top image shows the starting position for the patient on the patient support, slightly before a surview scan.

For the acquisition of the surview scan, the patient is moved into the scanner as shown in the middle image.

Thereafter, the patient support is (partially) retraced from the bore and moved into a starting position for the subsequent diagnostic scan as shown in the bottom image. Even in this camera view, a large part of the target region is visible and can be used to track relevant patient movement.

The invention is based on comparing images such as the top and bottom images shown in Fig. 2, to detect movement of the patient between the times just before the surview scan and just before the diagnostic scan. In a simplest implementation, the images chosen for comparison are at the same patient support position, More generally, patient movement is assessed between first and second scans, and these could both be diagnostic scans of an overall scan workflow rather than a surview scan and a diagnostic scan.

In a most simple implementation, the movement detection is used to generate a simple warning, but in more advanced implementations an automated adjustment of the scan plan may be implemented.

To check for patient movement, a pair of images is required for comparison. The comparison is made prior to the acquisition of the second scan (e.g., the diagnostic scan) between the camera image at that time and a previous camera image taken prior to the acquisition of the first scan (e.g., the surview), and with the same patient support table position.

In order to select first and second camera images at the same patient support position, the table movement may be tracked and the camera images may be labelled accordingly through the entire workflow. This tracking may be via patient support sensors, or by camera image analysis.

It is noted that the implementation of the invention only requires images to be captured at the same patient support position just before the two scans. Thus, there may only be two images taken, and they are both "selected". However, in practice, the camera is more preferably used for continuous patient monitoring, and the system selects the first and second camera images from a large set of camera images, which are used for other purposes additional to the motion identification of the invention. Thus, the movement sensing of the invention is an additional function to existing image analysis used by the scanner.

There are various known approaches for analyzing a pair of images to detect, and preferably also locate, motion between those images and to visualize such differences.

A first approach is to determine differences between images and use image overlay techniques for the visualization. The location of movement may thereby be identified.

A difference image obtained by image subtraction may for example be displayed on a display monitor (e.g. to the operator and/or to the subject) in an overlay, e.g. as a color overlay. Thus, differences of the currently observed state with respect to a previously observed state of the subject can be highlighted, e.g. by color to mark the differences. It will be understood that this may also be achieved by different types of overlay, such as changing the image intensity instead of color (e.g. literally highlighting differences), or creating a contour overlay to indicate a contour of the difference region. For example, a perimeter of an image region where the image difference exceeds a predetermined threshold may be marked by a line, e.g., a solid or dashed line.

A second approach is to provide a measure of image similarity. Examples of image similarity measures are Mean Square Error, Structural Similarity Index and Cosine Similarity. Depending on the method, a high or low score is indicative of changes in the image and hence patient motion. The score may then be used to trigger an alarm for the operator. A difference measure between images may comprise a maximum absolute difference, a mean absolute difference, a predetermined percentile of the (e.g. absolute) difference, a sum of squares, and/or any other such value to characterize a magnitude of the overall displacement between images.

The first and second camera images may be processed to find a spatial transformation that maximizes the image similarity measure. This spatial transformation is then representative of the movement between the images or image portions.

A third approach is to provide an optical flow and registration analysis. This approach establishes a mapping between the two images at pixel level. Hence, this method can be used not only to detect motion in general (e.g., using the average norm of the flow), but it could also be used to propagate planning information from one image to another.

A fourth approach is to use landmark-based methods: Using machine and deep learning-based techniques, patient landmarks (e.g., joints) can be detected. Similarly to optical flow analysis, landmark displacement can be used as an indicator for patient movement (i.e., to trigger an alarm), or to adjust the planning information.

For example, one or more specific image features may be detected (corresponding to anatomical features and/or landmarks), and movement may be detected and/or quantified based on displacement (and/or change of another spatial variable or variables, e.g. orientation) of such image features. For example, a change of position of the anatomical features and/or landmarks in the later image relative to the earlier image may be determined and/or spatial data relative to the position of the same feature and/or landmark.

When landmark features can be detected, the detection of movement may take changes in position of such landmark into account to be more sensitive to relevant motion as opposed to any arbitrary change in image content. It is also to be noted that such landmark features may be used to easily add 3D information to a 2D representation, e.g. a 2D (image) representation may easily show displacement of the subject in the image plane (e.g. by accenting the change with respect to the reference using a color overlay), while the landmark position may also be indicated on the visual representation together with an indicator that shows the depth (out-of-plane position) of the image feature and/or its relative change with respect to the earlier image.

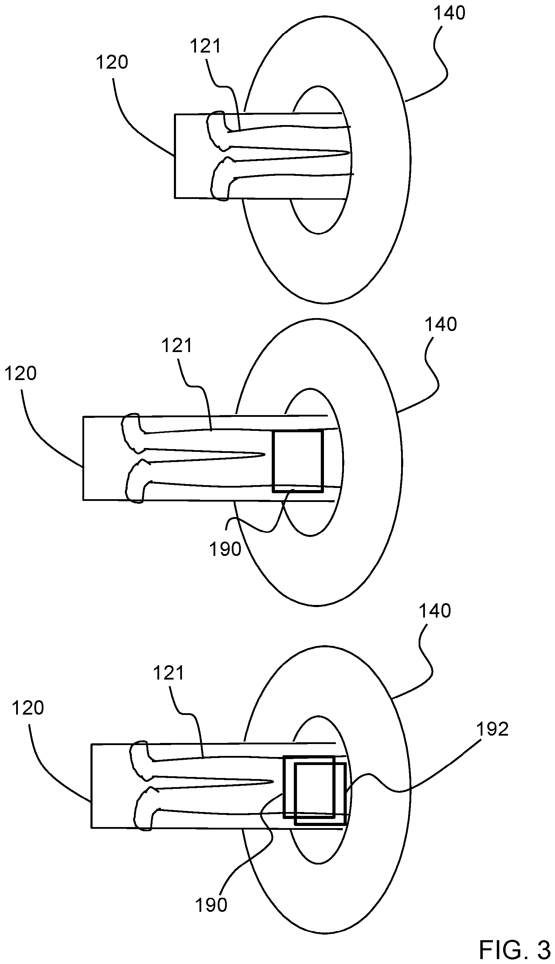

Fig. 3 shows schematically an adaption of the scan plan. The top image shows the surview scan.

The scan region for the diagnostic scan is planned from the results of the surview scan. The scan region is indicated in the middle image by rectangle 190, and it is backpropagated from the planning step using the surview data.

Based on the movement analysis (by which the scan region is transferred to an earlier frame of the image sequence corresponding to the starting position of the diagnostic scan), an adapted scan region 192 is derived.

The invention may be implemented using one or more optical or depth cameras. The camera or cameras may be outside the scanner bore as shown, but equally the camera may be in the scanner gantry, such that anatomies of interest are visualized.

Many existing medical scanners are equipped with a camera at the ceiling, so that the implementation of the system described can be achieved with only software components. Many existing medical scanners also use in-bore cameras, and again these cameras could also be used to detect and visualize patient motion between scans, such as between surview images and diagnostic scans.

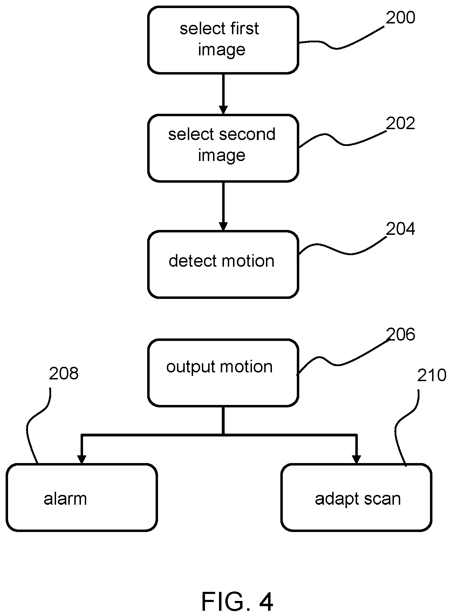

Fig. 4 shows a method for monitoring a patient during a medical scan.

In step 200, a first camera image is selected before a first scan.

In step 202, a second camera image is selected position after the first scan and before a second scan.

In step 204, the first and second camera images are processed to detect patient movement between the first and second camera images.

In step 206, an indication of the patient movement is output.

This output may be used simply to trigger an alert or alarm as shown by step 208, and/or it may be used to adapt the scanning plan of the second scan as shown in step 210.

Some implementations may simply detect any movement and hence raise an alarm. More sophisticated implementations may identify the regions at which movement has occurred and determine if the motion includes the region of interest being scanned. The adaptation of scan plan for example additionally requires an amount and direction of movement to be assessed so that the scan plan can be adapted accordingly, such that the second (diagnostic) scan correctly correlates with the previous first (surview) scan.

The invention may be applied to any medical scanner in which a patient support is moved during the imaging procedure, like a PET imaging device, an MR imaging device, a SPECT imaging device, as well as a CT scanner as described above. The medical scanner may comprise a C-arm or a closed bore. The camera or cameras may be located in the bore or at an inner surface of the C-arm, or outside the bore or C-arm envelope.

The camera is static relative to the main body of the scanning system and thus the patient support moves relative to the camera whereas the fixed background is static between the camera images. The camera may not be mounted directly to the medical scanner but may instead be in a fixed position relative to the medical scanner by a separate mounting.

Although in the above described embodiments the patient support is always a patient support on which a patient is lying during the acquisition of the medical image, the patient support can also be configured for a sitting or a standing patient.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

Functions implemented by a processor may be implemented by a single processor or by multiple separate processing units which may together be considered to constitute a "processor". Such processing units may in some cases be remote from each other and communicate with each other in a wired or wireless manner.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to". If the term "arrangement" is used in the claims or description, it is noted the term "arrangement" is intended to be equivalent to the term "system", and vice versa.

Any reference signs in the claims should not be construed as limiting the scope.

## Claims

1. A system for monitoring a patient during a medical scan, wherein the medical scan involves moving the patient on a patient support relative to a scanning gantry, the system comprising:
a camera (130) for generating camera images of the patient on the patient support; and
a processor (150) for processing the camera images, wherein the processor is configured to:
(160) select a first camera image before a first scan;
(162) select a second camera image after the first scan and before a second scan;
(164) process the first and second camera images to detect patient movement between the first and second camera images; and
output an indication of the patient movement.

2. The system of claim 1, wherein:
the first and second camera images are taken with the patient support at a same patient support position; or
the first and second camera images are taken with the patient support at different patient support positions and processing the first and second camera images takes into account the patient support position.

3. The system of claim 1 or 2, wherein the first scan comprises a surview scan and the second scan comprises a diagnostic scan.

4. The system of claim 1 or 2, wherein the processor (150) is configured to:
receive an input indicating the patient support position; or
analyze the camera images to track patient support movement and thereby determine the patient support position.

5. The system of any one of claims 1 to 4, wherein the processor (150) is configured to process the first and second camera images by registering the images to detect differences.

6. The system of any one of claims 1 to 4, wherein the processor (150) is configured to process the first and second camera images by finding a spatial transformation that maximizes an image similarity measure.

7. The system of any one of claims 1 to 4, wherein the processor (150) is configured to process the first and second camera images by performing a flow analysis between the images or parts thereof.

8. The system of any one of claims 1 to 4, wherein the processor (150) is configured to process the first and second camera images by detecting landmark positions in the images.

9. The system of any one of claims 1 to 8, wherein the indication of the patient movement comprises an alarm.

10. A medical scanner comprising;
a patient support (120);
a scanning gantry (140);
a drive system (124) for moving the patient support relative to a scanning gantry in a patient movement direction;
a scan controller (126) for controlling the medical scanner to implement a scanning plan; and
the system of any one of claims 1 to 9.

11. The system of claim 10, wherein the scan controller (126) is configured to modify the scanning plan based on the indication of patient movement.

12. A method for monitoring a patient during a medical scan, wherein the medical scan involves moving the patient on a patient support relative to a scanning gantry, the method comprising:
(200) selecting a first camera image before a first scan;
(202) selecting a second camera image after the first scan and before a second scan;
(204) processing the first and second camera images to detect patient movement between the first and second camera images; and
(206) outputting an indication of the patient movement.

13. The method of claim 12, comprising receiving an input indicating the patient support position or analyzing the camera images to track patient support movement and thereby determine the patient support position.

14. The method of claim 12 or 13, comprising processing the first and second camera images by:
registering the images to detect differences; or
finding a spatial transformation that maximizes an image similarity measure; or
performing a flow analysis between the images or parts thereof; or
by detecting landmark positions in the images.

15. A computer program comprising computer program code which is adapted, when said program is run on a computer, to implement the method of any one of claims 12 to 14.
